(19) Europäisches Patentamt
European Patent Office
Office européen des brevets

(11) EP 1 486 505 A1

(12) EUROPEAN PATENT APPLICATION

(43) Date of publication:
15.12.2004 Bulletin 2004/51

(51) Int Cl.[7]: **C07K 7/14**, C07K 14/00, A61K 47/48, C12N 15/16

(21) Application number: **04016314.9**

(22) Date of filing: **23.06.1998**

(84) Designated Contracting States:
**AT BE CH CY DE DK ES FI FR GB GR IE IT LI LU MC NL PT SE**

(30) Priority: **24.06.1997 GB 9713361**
**23.04.1998 GB 9808696**

(62) Document number(s) of the earlier application(s) in accordance with Art. 76 EPC:
**98930927.3 / 1 001 978**

(71) Applicant: **Proteus Molecular Design Limited**
**Macclesfield, Cheshire SK11 0JL (GB)**

(72) Inventors:
- **Glover, Francis James**
  **Congleton Cheshire CW12 3DS (GB)**
- **Rushton, Arthur**
  **Wilmslow Cheshire SK9 2BE (GB)**
- **Morgan, Phillip John**
  **Congleton Cheshire CW12 3RP (GB)**
- **The other inventors have agreed to waive their entitlement to designation.**

(74) Representative: **Cockbain, Julian, Dr.**
**Frank B. Dehn & Co.,**
**European Patent Attorneys,**
**179 Queen Victoria Street**
**London EC4V 4EL (GB)**

Remarks:
This application was filed on 12.07.2004 as a divisional application to the application mentioned under INID code 62.

# (54) Angiotensin derivatives

(57) An angiotensin derivative comprising at least one angiotensin peptide moiety coupled to a peptide carrier-binding moiety which can be used for therapy and prophylaxis of conditions associated with the renin activated angiotensin system.

EP 1 486 505 A1

## Description

The present invention relates to analogues or derivatives of the mammalian peptide hormones angiotensin I and angiotensin II, and to immunotherapeutic uses of these in particular for the therapy and prophylaxis of conditions associated with the renin activated angiotensin system.

Angiotensin peptides are involved in controlling arterial pressure in mammals. They are produced in several forms in the body as a result of a biochemical cascade known as the renin-angiotensin system (RAS), initiated by renin produced as a result of a fall in arterial pressure. In the RAS, represented schematically below, renin is released by the kidneys from stored pro-renin following a fall in arterial blood pressure, and acts enzymatically upon angiotensinogen to produce angiotensin I which is a decapeptide having the sequence Asp-Arg-Val-Tyr-Ile-His-Pro-Phe-His-Leu. Two amino acids from the C-terminus of angiotensin I are rapidly cleaved, by angiotensin converting enzyme (ACE), present in the endothelium of the lungs, generally within 1-2 seconds, to produce the octapeptide angiotensin II, having the sequence Asp-Arg-Val-Tyr-Ile-His-Pro-Phe.

```
Angiotensinogen ----------> angiotensin I-----> angiotensin II
                     ↑                       ↑
                   renin      angiotensin converting enzyme (ACE)
```

Angiotensin I is very short lived within the body and has mild vasoconstrictor activity. Alone therefore it has insignificant effect on the circulatory system. Angiotensin II, however, is a vasoactive peptide which has a profound effect on the circulatory system, as well as on the endocrine system. Elevated levels of RAS-activated angiotensin II cause vasoconstriction and renal retention of salt and water, both of which contribute to increased arterial pressure (hypertension) which can lead to cardiovascular damage. Angiotensin II has been implicated in a number of other disease states, including congestive heart failure. Hypertension is a major risk factor for heart attacks and strokes and congestive heart failure is the disease with the highest mortality within a few years of onset. There is a need for effective therapies for combatting these and other diseases associated with the renin-angiotensin system.

Current treatment for these diseases includes intervention in the RAS system using small organic molecules. One approach attempts to inhibit ACE with inhibitors such as lisinopril, captopril and enalapril, agents which are now established in management of hypertension. These drugs have not however been entirely successful. It seems that inhibition of ACE is only partial. Furthermore, because ACE lacks substrate specificity, biotransformation of other metabolically active peptides, including bradykinin may also be inhibited, which is undesirable. In addition, these drugs need to be taken on a regular basis, often for long periods, such as for the majority of adult life. A major drawback, however, of these drugs is their undesirable side effects, including dry cough and a first dose hypotensive effect with dizziness and possible fainting. Since anti-hypertensive therapies invariably need to be taken long term, e.g. for up to 30 years and sometimes even longer, these adverse side effects can result in loss of patient compliance, particularly in the absence of short term clinical benefit in a mainly asymptomatic condition, severely limiting the usefulness of this therapeutic approach.

A more recent therapeutic approach involves drugs which are angiotensin receptor antagonists which are intended to block the activity of angiotensin II. Examples include losartan and valsartan. The agents which have been developed to date appear to be specific for only the $AT_1$ angiotensin receptor; they therefore block the dominant vasoconstrictor effects of angiotensin II, and are better tolerated but do not affect other actions of the angiotensin hormones. Experience with $AT_1$ receptor antagonists indicates that whilst they may be of comparable effectiveness to ACE inhibitors poor patient compliance remains a problem. There is accordingly a need for improved therapies of diseases associated with the RAS.

A potential approach in treating or preventing diseases or disorders associated with the activity of a hormone is to neutralise the effects of the hormone within the patient by immunotherapy i.e. by immunising the patient against the hormone such that the activity of the hormone is neutralised by specific anti-hormone antibodies. Such antibodies may be exogenously administered in passive immunisation or they may be generated *in situ* by active immunisation using an immunogen based on the hormone.

We have now developed new derivatives of angiotensin which are potent immunogens and which can be used in an immunotherapeutic approach to combat conditions associated with elevated levels of angiotensin II produced by the RAS.

In particular, derivatives of angiotensin have been developed in which one or more angiotensin peptides are coupled to a binder moiety, e.g. a peptide sequence, which facilitates attachment of the angiotensin peptide to an immunological carrier such as a protein or polypeptide to form an immunogenic conjugate capable in an immunised host of inducing antibodies which bind to angiotensin and neutralise its effects. These induced antibodies include those

which may also bind to the precursor form, angiotensinogen and in this way, cleavage by renin to angiotensin I is prevented, thereby providing an additional blockade of the system. This may be particularly relevant to reducing the effects of modulation of the negative feedback effects of Angiotensin II on renin production and release of Angiotensin I.

In one aspect, the present invention thus provides an angiotensin derivative comprising at least one angiotensin peptide moiety coupled to a peptide carrier-binding moiety.

These angiotensin derivatives may be used to immunise a patient against the hormone angiotensin II and/or its polypeptide precursor angiotensin I and/or angiotensinogen such that the activity of the hormone is neutralised by specific anti-hormone or antipolypeptide antibodies.

The angiotensin peptide moiety may be any moiety, without necessarily having the biological activity of a native angiotensin (ie. native hormone activity at the receptors, including both angiotensins I and II), in the body which is capable of acting as an immunomimic of native angiotensin peptides i.e. which immunologically mimics angiotensin so as to generate antibodies which bind to native angiotensin peptides. Thus, such a moiety may conveniently comprise an angiotensin peptide, preferably angiotensin I (a decapeptide of formula Asp-Arg-Val-Tyr-Ile-His-Pro-Phe-His-Leu) or angiotensin II (an octapeptide of formula Asp-Arg-Val-Tyr-Ile-His-Pro-Phe), or a functionally equivalent variant thereof. Such variants may include modifications of the angiotensin I or II sequence by single or multiple amino acid substitution, addition or deletion and also sequences where the amino acid residues are chemically modified, but which nonetheless retain angiotensin immunogenic activity. Such functionally (ie. immunologically) equivalent variants may occur as natural biological variations, or they may be prepared using known and standard techniques for example by chemical synthesis or modification, mutagenesis, e.g. site-directed or random mutagenesis etc. The important feature as regards the modification is that the angiotensin peptide retains the ability to act as immunomimic of native angiotensin. Thus for example, an amino acid may be replaced by another which preserves the physicochemical character of the angiotensin peptide or its epitope(s) e.g. in terms of charge density, hydrophilicity/hydrophobicity, size and configuration and hence preserve the immunological structure. "Addition" variants may include N- or C-terminal fusions as well as intrasequence insertion of single or multiple amino acids. Deletions may be intrasequence or may be truncation from the N- or C-termini. The term "angiotensin peptide" as used herein includes all native angiotensin peptides and their functionally equivalent variants.

The carrier-binding moiety serves as a means by which the angiotensin peptide moiety may be attached to an immunological carrier, which will generally be a protein or polypeptide, and thus preferably contains an amino acid residue having a reactive side chain, via which the angiotensin derivative may readily be coupled to the carrier using standard coupling techniques. Advantageously such a side chain may contain a free hydroxyl, carboxyl or thiol group. Such an amino acid may thus conveniently be a cysteine, tyrosine, aspartic acid or glutamic acid residue or a derivative thereof such as N-acetyl cysteine.

Angiotensin analogues of the invention have been shown to have improved coupling to an immunological carrier for inducing antibodies which can be used immunotherapeutically and these analogues have advantages in this regard over the native peptide.

The carrier-binding moiety may take the form of a peptide extension at the N- or C-terminal of an angiotensin peptide, or a peptide pendant from or disposed within a chain segment between two or more angiotensin moieties.

Viewed from a further aspect, the present invention can be seen to provide an angiotensin derivative of Formula I

$$((A)\text{-}X_n)_m\text{-}L_p\text{-}Y\text{-}[L_q\,(X_r\text{-}(A))_s]_t \qquad (I)$$

wherein

A represents an angiotensin peptide moiety;
X represents an amino acid;
Y represents an amino acid having a side chain with a free -SH, -OH or -COOH group;
L represents an organic linker capable of binding a group ((A)-$X_n$)- at one or more sites, e.g. capable of binding up to 10 (A)$X_n$ moieties;
n and r are each = 0-20;
m and s are each ≥ 1, e.g. 1 to 10, preferably 1, 2, 3 or 4; and
p, q and t are each 0 or 1;

with the proviso that if m≥2, then p=1, or if s≥2, then q=1.

Preferably A is an angiotensin peptide, and X may be attached at the N- or C-terminus of the angiotensin peptide.

Group L may be any organic linker structure, preferably however, it is a peptide chain, which may be linear or branched or a single amino acid residue, containing residues of natural or synthetic amino acids or pseudo-amino acids. However it may also represent a carboxyl- or amine-terminating dendritic or cascade polymer, for example a branched polyamine.

When t=0, it will be seen that the compounds of Formula (I) include derivatives wherein a carrier binding moiety (i.e. X-Y or X-L-Y) is attached at the Nor C-terminus of an angiotensin peptide, as a simple Nor C-terminal extension, or wherein multiple angiotensin peptide moieties are linked to a carrier-binding moiety terminating in a group Y, for example as a dendritic array or where the angiotensin moieties are attached at multiple sites on the carrier-binding moiety.

When t=1, it will be seen that the derivatives may take the form of a "dimer"-type structure wherein the carrier-binding group Y of the carrier-binding moiety is disposed within a chain segment of the derivative i.e. effectively between two or more angiotensin peptide moieties.

If t=1, and L is an amino acid residue or a peptide chain, L may be or include a "chain-inverting" amino acid or pseudo amino acid (i.e. a compound capable of linking two peptide moieties, e.g. a diamine or dicarboxylic acid), this being a compound capable of inverting or reversing the N- to C-terminal direction of the peptide chain. Such a compound will thus generally include two amino or two carboxylic acid groups, e.g. glutamic acid or a $\alpha,\omega$-alkylene diamine or $\alpha,\omega$-alkylene dicarboxylic acid. When t=1, it is furthermore preferred that the total number of groups ((A) $-X_n$) - does not exceed 8.

Preferred compounds of Formula (I) include those wherein n and r are each 0-10, preferably 1-6, and those wherein m and s are each ≤8, preferably 1, 2 or 4.

Group X preferably represents an amino acid having no side chain or a hydrocarbyl side chain (preferably an alkyl, $C_{3-7}$ cycloalkyl or cycloalkenyl, $C_{3-7}$ cycloalkyl- or cycloalkenyl-alkyl, alkaryl, aralkyl or alkarylalkyl moiety in which each alkyl moiety may be saturated or unsaturated and contains up to 6 carbons and each aryl moiety is preferably a phenyl ring), particularly preferably an aliphatic side chain. Glycine, alanine, β-alanine, valine, leucine and isoleucine are preferred and glycine is especially preferred.

Group Y is preferably cysteine, tyrosine, glutamic acid or aspartic acid or a derivative thereof such as N-acetyl-cysteine.

Group L preferably contains at least one residue of an amino acid or pseudo amino acid containing at least two amine or carboxyl groups e.g. lysine, arginine, glutamic acid or aspartic acid, particularly where t=0. Conveniently, such a preferred group L is a linear or branched peptide chain, eg. containing 2 to 15 amino acid residues. Branching may, of course, occur by peptide bond formation at an amine or carboxyl group of an amino acid residue side chain, eg. at a side chain amine group of lysine or arginine or at a side chain carboxyl group of aspartic or glutamic acid. A group L comprising one or more, eg. 1 to 3, lysine residues is especially preferred. Branching may occur by peptide bond formation at both the $\alpha$-amino and $\varepsilon$-amino groups of lysine.

Preferred compounds of Formula (I) thus include compounds of Formulae (II) to (IV):

$$(A)\text{-}X_n\text{-}Y \qquad (II)$$

$$(A)\text{-}X_n\text{-}L\text{-}Y \qquad (III)$$

$$((A)\text{-}X_n)_m\text{-}L\text{-}Y \qquad (IV)$$

$$(A)\text{-}X_n\text{-}L\text{-}Y\text{-}L\text{-}X_r\text{-}(A) \qquad (V)$$

wherein A, X, L, n and r are as hereinbefore defined and m ≥ 2.

Where the compounds of Formula (IV) contain more than one (A) group, these are preferably attached at the same terminus i.e. preferably all are N-terminally or all are C-terminally attached.

In compounds of Formulae (II) and (III) where X is C-terminally attached to a group A being an angiotensin peptide, Y is preferably cysteine. Where X is attached to the N-terminus of A, Y is preferably N-acetyl-cysteine.

In Formulae (II) to (V) , $X_n$ or $X_r$ are each preferably chains of 1 to 6 glycine residues.

In compounds of formula (IV), m is preferably 2 or 4.

In Formulae (III) to (IV), L is preferably lysine, $-lys-(X)_u$, $-lys-lys-(X)_u$, or

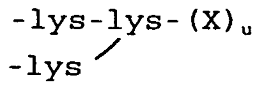

wherein u is 0 to 10, preferably 0 to 6, and X is an amino acid as defined above.

Thus, preferred compounds of Formula (IV) are those of Formulae (VI) and (VII):

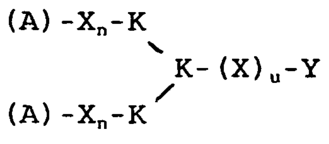

(VI)

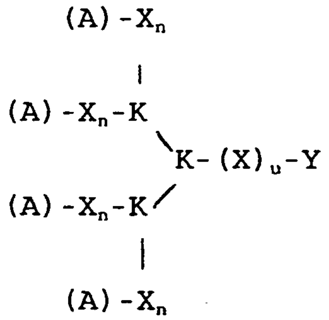

(VII)

where A, X, Y, n and u are as hereinbefore defined, and K is lysine.

In the "dimer-type" derivatives of Formula (V) the angiotensin peptide moiety may preferably be a "reversed" or "inverted" sequence variant of an angiotensin peptide ie. an angiotensin peptide in which the order of the constituent amino acids is reversed.

Representative angiotensin derivatives according to the invention include:

A- $(Gly)_{1\text{-}6}$-cys;

N-Acetylcys- $(Gly)_{1\text{-}6}$-A;

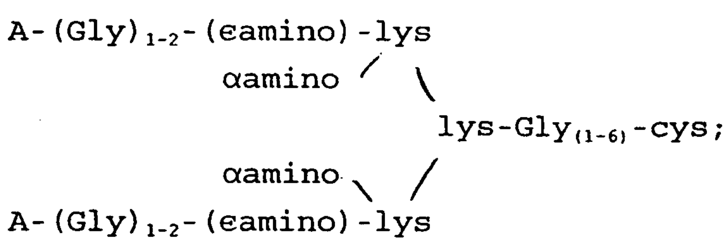

(The A-$(Gly)_{1\text{-}2}$-moiety may be bonded to either the α-amino or the ε-amino group)

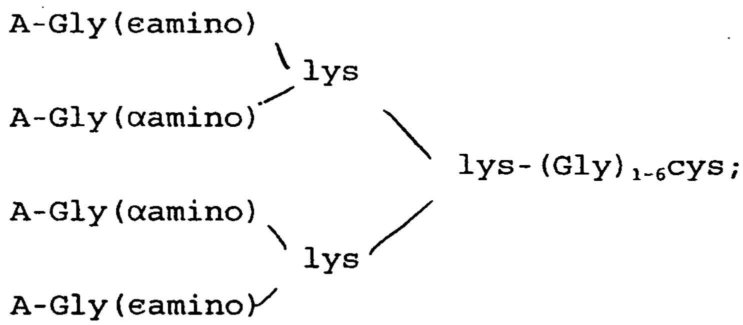

and

A'- $(Gly)_{1-6}$-cys- $(Gly)_{1-6}$-A;

N-acetyl-Cys-Ala-Angiotensin

N-acetyl-Cys-$(Ala)_4$-Angiotensin

N-acetyl-Cys$(Gly)_6$-Angiotensin

N-acetyl-Cys-Gly-Ala-Gly-Ala-Angiotensin

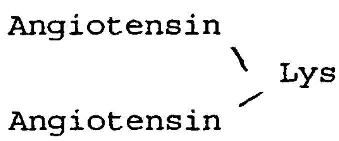

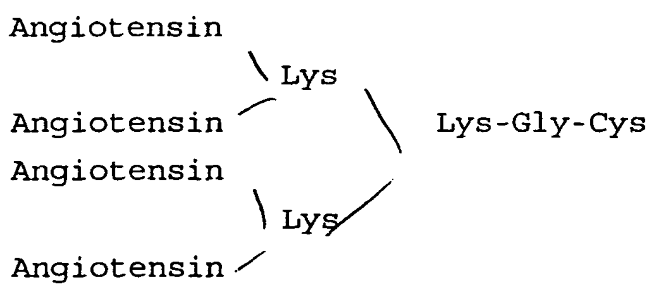

(A)-Gly Cys

(A)-Cys

(A)-Tyr

N-acetyl-Cys-(A)

Tyr-(A)

N-acetyl-Cys-Gly-(A)

Cys - (A)

wherein A is angiotensin I or angiotensin II and A' is angiotensin I or angiotensin II or an inverted or reverse angiotensin I or angiotensin II sequence.

Although Glycine is preferred, aliphatic side chain amino acids may be used in place of one or more of the Gly residues in the above formula.

Although the peptide analogues of the invention when examined by computer-aided energy minimisation modelling are generally considered too small to be optimally immunogenic alone, it has been found that when coupled via the carrier-binding moiety to a carrier, these peptide analogues elicit a strong protective immune response. They are thus eminently suitable for use in immunotherapy against RAS-associated conditions. Without wishing to be bound by theory, it is believed that coupling of the peptides to a carrier by means of the carrier-binding moiety results in the analogues having substantially the same conformation as that of the native angiotensin peptides.

The new derivatives according to the invention may be generated using a number of standard techniques including, for peptides, the Merrifield solid phase method in which amino acids are added stepwise to a growing polypeptide linked to a solid matrix as described in R.B. Merrifield, Fed. Proc. Amer. Soc. Biol. (1962). 21, 412 and R.B. Merrifield, Jour. Amer. Chem. Soc. (1963), 85, 2149 and conventional FMOC chemistry. If desired, reactive side chain groups of the amino acids in the growing chain may be protected during the chain synthesis. Branched structures may be prepared by similar techniques.

Where the new derivatives are linear peptides these may also be prepared by recombinant DNA expression using techniques known in the art e.g. as described, for example, by Sambrook et al., in Molecular Cloning: A Laboratory Manual, Second Edition, 1989.

Thus the present invention also provides a nucleic acid molecule coding for the angiotensin peptide derivatives of the invention, and nucleic acid molecules with sequences complementary thereto.

According to a further aspect of the invention, we provide an expression vector comprising the said nucleic acid molecule of the invention. Such a vector may be suitable for expression in microorganisms which may be prokaryotic or eurkaryotic e.g. *E coli* or yeast, or in plant or animal e.g. mammalian cells.

Such an expression vector, capable *in situ* of synthesising an angiotensin derivative according to the invention may also be used therapeutically and may be introduced to the subject in a variety of ways. Examples of these include topical application of the 'naked' nucleic acid vector in an appropriate vehicle for example in solution in a pharmaceutically acceptable excipient such as phosphate buffered saline (PBS), or administration of the vector by physical methods such as particle bombardment, also known as 'gene gun' technology, according to methods known in the art e.g. as described in US-5371015 in which inert particles, such as gold beads coated with the vector are accelerated at speeds sufficient to enable them to penetrate the skin surface, by means of discharge under high pressure from a projecting device.

Nucleic acid sequences encoding angiotensin derivatives of the invention may also be used immunotherapeutically in the form of delivery vectors. These include viral delivery vectors, such as adenovirus or retrovirus delivery vectors known in the art into which the nucleic acid sequence is incorporated and which can be used for immunisation in ways known in the art.

Other non-viral delivery vectors which may be used to deliver the nucleic acid vectors of the invention include lipid delivery vectors, including liposome delivery vehicles, known in the art.

According to a yet further aspect, the present invention provides a host organism transformed with a vector according to the invention.

The angiotensin derivatives of the invention, as is the case for other small molecules, may be of insufficient size to stimulate antibody formation alone and may thus need to be conjugated to a macromolecular carrier in order to stimulate antibody production and a protective immune response.

Thus according to a further aspect, the present invention provides an angiotensin derivative as defined above conjugated to a carrier, preferably a polypeptide carrier.

Coupling of the derivative of the invention to the carrier may be by methods known in the art for example by treatment with heterobifunctional linking agents. Where coupling is via a terminal cysteine (or N-acetyl cysteine), the linking agent may be m-Maleimidobenzoyl-N-hydroxysulphosuccinamide ester; in which case maleimide modifies one or more lysine side chains in the peptide carrier, and a thioether bond forms at the terminal cysteine residue. Other coupling reagents known in the art, eg carbodiimide coupling, may also be used.

Any carrier known in the art for such purposes may be used, including the purified protein derivative of tuberculin, tetanus toxoid, diphtheria toxoid, keyhole limpet haemocyanin or derivatives thereof.

Where the angiotensin derivative is a linear peptide and the carrier is a protein or polypeptide, the entire peptide conjugate may also be made by recombinant DNA methods wherein a nucleic acid molecule encoding the

conjugated molecule is expressed in an appropriate host cell.

The new angiotensin derivatives of the invention may be used in an immunotherapeutic approach to combatting diseases associated with normal or elevated levels of RAS activity and/or angiotensin peptides, and represents an advantageous method compared to currently available methods. Patient compliance should be increased in that less frequent dosing than is the case with current therapies is involved, and undesirable side effects are avoided.

Thus according to a further aspect, the present invention provides a pharmaceutical composition comprising an angiotensin derivative according to the invention, or a conjugated angiotensin derivative according to the invention, together with one or more pharmaceutically acceptable carriers or excipients.

Viewed from a further aspect, the invention provides an angiotensin derivative according to the invention for use in therapy.

Viewed from a yet further aspect, the invention provides the use of an angiotensin derivative according to the invention in the manufacture of a medicament for use in combatting diseases associated with the renin-angiotensin system. Such diseases include congestive heart failure and hypertension such as systemic hypertension and other diseases in which the renin-angiotensin system contributes to the pathophysiology thereof, as well as diseases where the renin-angiotensin system has elevated levels of activity.

Viewed from a still yet further aspect, the invention provides a method of combatting conditions associated with the renin-angiotensin system comprising administering an angiotensin derivative according to the invention.

The method may be used to modulate blood pressure.

The angiotensin derivative according to the invention optionally conjugated to a carrier or recombinant nucleic acid encoding for the derivative may be administered by all conventional methods including parenterally (e.g. intraperitoneally, subcutaneously, intramuscularly, intradermally for example in the form of inert particles such as gold pellets or beads to which the derivative is adsorbed which may be accelerated at speeds sufficient to enable them to penetrate the skin of a subject, or intravenously), topically (e.g. as a cream to the skin), intra-articularly, mucosally (e.g. orally, nasally, vaginally, rectally and via the intra-ocular route) or by intrapulmonary delivery for example by means of devices designed to deliver the agents directly into the lungs and bronchial system such as inhaling devices and nebulisers, and formulated according to conventional methods of pharmacy optionally with one or more pharmaceutically acceptable carriers or excipients, such as for example those described in Remingtons Pharmaceutical Sciences, ed. Gennaro, Mack Publishing Company, Pennsylvania, USA (1990).

Such compositions are conveniently formulated in unit dosage form e.g. for mucosal, parenteral or oral administration.

Actual treatment regimes or prophylactic regimes, formulations and dosages will depend to a large extent upon the individual patient and may be devised by the medical practitioner based on the individual circumstances.

The type of formulation will be appropriate to the route of administration. For example, parenteral administration by subcutaneous or intramuscular injection may be with a sterile aqueous suspension of the conjugated analogue in PBS, saline or water for injection, optionally together with one or more immunological adjuvants e.g. aluminium hydroxide, saponin, quil A, muramyl dipeptide, mineral or vegetable oils, vesicle-based adjuvants, non-ionic block copolymers, or DEAE dextran. Additional components such as preservatives may be used.

The dosage for injection may be in the range 1-100 μg peptide equivalent and the frequency of administration may be upwards of from once every three or six months, to once every year or once every five years.

For oral administration, the conjugated derivatives may be formulated as tablets, liquid, capsules etc. Dosages range from 1 to 1000 μg peptide equivalent with dosing occurring at intervals dependent on bioavailability of product.

According to a still yet further aspect, the present invention provides a method for achieving maximal blockade of angiotensin hormones comparable to or exceeding that achieved by existing therapies based on ACE inhibitors and/or angiotensin II receptor antagonists, said method comprising administering an angiotensin derivative according to the invention.

The invention will now be described in further detail in the following non-limiting Examples, with reference to the drawings in which:

Figure 1 is a graph showing antibody titres +/sem, n=6 (dilution corresponding to 0.1 increase in OD) against time (sample day);

A = Control
B = Derivative 3 of Example 2
C = Derivative 1 of Example 2
D = Derivative 4 of Example 2
E = Derivative 2 of Example 2.

Figure 2 is a graph showing peak change in blood pressure following administration of AI in control rats and in rats

immunised with a conjugate of an analogue of AI in groups C and J of Example 4.

Figure 3 shows recordings of mean blood pressure changes in response to AI in animals of groups A and C of Example 4.

Figures 4, 5 and 6 are bar charts showing antibody titres measured in terms of $A^{450}$ in an ELISA assay using in the assay in Figure 4 angiotension I, in Figure 5 angiotensin II and in Figure 6 angiotensinogen, the ELISAs showing the binding of partially purified rat antisera raised against vaccines containing analogues of angiotensin hormones.

Figures 7 and 8 are graphs showing antibody titres against time (sample day) for the following derivatives

N-acetyl-Cys-$(Ala)_4$-Angiotensin I
N-acetyl-Cys$(Gly)_6$-Angiotensin I

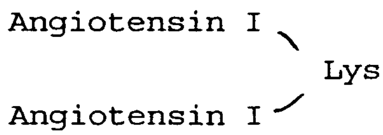


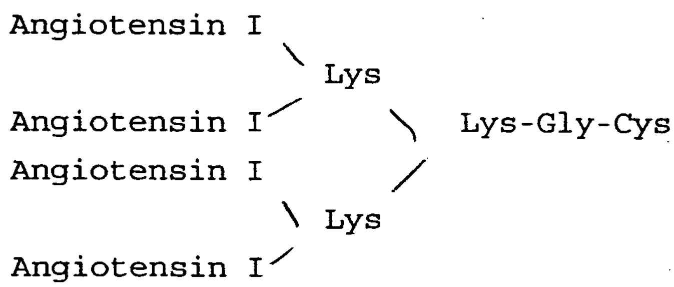


**Example 1:** Peptide generation.

Peptides were synthesised by the Fmoc strategy of solid phase peptide synthesis on a Protein Technologies, Symphony Peptide Synthesiser. The resin used was Tentagel S-NH2 with a Rink Amide linker. The side chain protecting groups of the Fmoc amino acids used were Trt for Cys His, Asn and Gln, tBu for Tyr Thr, Asp, Glu and Ser; Boc for Lys and the indole N of Trp, Pmc for Arg. Activation of the carboxyl groups was achieved using, TBTU/HOBt/DIPEA, all couplings were carried out in DMF. Deprotection of the Fmoc groups was achieved with 20% Piperidine in DMF. Cleavage of the peptides from the resin was carried out with 5%Anisole/5%Thioanisole/5%EDT/3%Water/2%TES in TFA for 1 hour. The peptides were purified by RP-HPLC using a 40mm x 210mm Deltapak C18 radial compression column on a Waters Deltaprep 4000 and characterised by MALDI-TOF on a Kratos Maldi 3 and by AAA.

For dendrimers Fmoc Lys(Fmoc)-OH is attached by the methods above and gives both $\alpha$ and $\varepsilon$ amino groups free for peptide elongation. Quantities of Fmoc amino acids used have to be increased accordingly.

Rink Amide Linker = p-[(R,S--[1-(9H-Fluoren-9-yl)-methoxyformamido]-2,4-dimethoxybenzyl]-phenoxyacetic acid

Fmoc =9-Fluorenylmethoxycarbonyl
Trt = Trityl, Triphenylmethyl
tBu = tertiary butyl
Boc = tertiary butyloxycarbonyl
Pmc = 2,2,5,7,8-Pentamethylchroman-6-sulphonyl
TBTU = 2-(1H-Benzotriazole-1-yl)-1,1,3,3-tetramethyluronium tetrafluoroborate
HOBt = N-Hydroxybenzotriazole
DIPEA = Diisopropylethylamine
DMF = N,N Dimethylformamide
EDT = Ethanedithiol

TES = Triethylsilane
TFA = Trifluoroacetic acid
RP-HPLC = Reverse phase high performance liquid chromatography
MALDI-TOF = Matrix assisted laser desorption ionisation - time of flight
AAA = Amino acid analysis
Fmoc-Lys(Fmoc)-OH = α, ε di-9-fluorenylmethoxycarbonyl lysine

The following peptides were synthesized in this manner:

| | |
|---|---|
| (1) Angiotensin I-gly-cys | Asp-Arg-Val-Tyr-Ile-His-Pro-Phe-His-Leu Gly-Cys |
| (2) Angiotensin II-gly-cys | Asp-Arg-Val-Tyr-Ile-His-Pro-Phe-Gly-Cys |
| (3) N-acetyl-Cys-Gly-Angiotensin I | N-acetyl-Cys-Gly-Asp-Arg-Val-Tyr-Ile-His-Pro-Phe-His-Leu |
| (4) N-acetyl-Cys-Gly-Angiotensin II | N-acetyl-Cys-Gly-Asp-Arg-Val-Tyr-Ile-His-Pro-Phe |

**Example 2:** Conjugation procedure.

To tetanus toxoid solution in phosphate buffered saline (PBS), a 60 molar excess of S-MBS, m-Maleimidobenzoyl-N-hydroxysulphosuccinimide ester is added and stirred for 2 hours at 4°C in a sealed vial.
Excess S-MBS crosslinker is removed by chromatography (gel exclusion, PD-10, G-25 sephadex column) in PBS. The activated tetanus toxoid peak is collected, assayed for free maleimido groups and used as below.
The resulting carrier protein solution is purged with $N_2$, and a 12 molar excess of angiotensin derivative peptide added. The resulting solution is stirred for 4 hours at 20°C in a sealed container.
The conjugate is purified from free peptide by gel exclusion chromatography as above. A final assay for loss of free maleimido groups is performed on a sample of the mixture to prove that all available sites are conjugated.
The final conjugate is diluted to a working concentration and formulated as desired.
The structure of S-MBS crosslinked conjugate with a linear C-terminal extended angiotensin peptide derivative (eg. derivatives (1) and (2) of Example 1) is shown below:

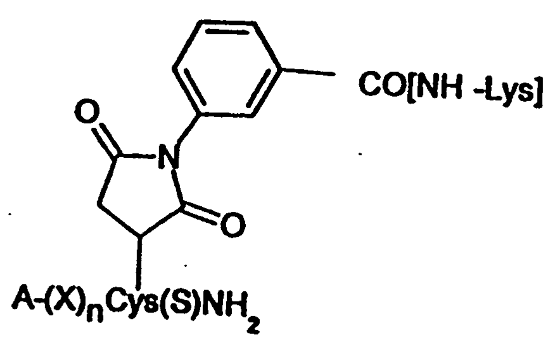

Following this procedure, angiotensin derivatives (1) to (4) of Example 1 were conjugated individually to aliquots of tetanus toxoid.

**Example 3:** Immunisation Studies.

The four angiotensin derivatives of Example 1, conjugated individually to aliquots of tetanus toxoid as described in Example 2, were formulated by adsorption to 0.4% (w/v) aluminium hydroxide gel (Alhydrogel, Superfos s/a, Denmark) in a normal saline (0.9% (w/v)) vehicle.

All conjugates were used as 10µg/ml peptide equivalent solution.

Male Sprague-Dawley rats were used in 5 treatment groups, with 6 rats per group.

The treatment groups received:

| | |
|---|---|
| Vehicle, sterile saline | 0.5 ml/rat |
| N-acetyl-cys-gly-Angiotensin I derivative immunotherapeutic | 5 µg peptide equivalent/ rat in 0.5ml vehicle |
| Angiotensin I-gly-cys derivative immunotherapeutic | 5 µg peptide equivalent/ rat in 0.5 ml vehicle |
| N-acetyl-cys-gly-Angiotensin II derivative immunotherapeutic | 5 µg peptide equivalent/ rat in 0.5 ml vehicle |
| Angiotensin II-gly-cys derivative immunotherapeutic | 5 µg peptide equivalent/ rat |

The route used was subcutaneous and each rat received 3 separate doses of the specified test article during the course of the study.

The bodyweight of each rat is recorded once a week throughout the experimental procedure.

**Experimental procedure**

In this initial investigation the core temperature of each rat was recorded, as part of the general physiological monitoring of the animals.

On day 1 and subsequently on days 22 and 43, the rats received a single subcutaneous dose of the vehicle, or the test articles. Twenty four hours following each administration (days 2, 23 and 44) and on further days specified in table 1 a venous blood sample (0.5 ml) was subsequently taken while the rat was restrained.

Each sample of venous blood was collected in a glass tube, cooled on ice and allowed to clot, then centrifuged to yield serum within 45 minutes of sampling. Serum samples were frozen at approximately -20°C as soon as possible.

Table 1:

| **Time Schedule of Study Procedures** | | | |
|---|---|---|---|
| **Week** | **Day** | **Treatment** | **Blood Sample** |
| **1** | 1 | Test articles, vehicle | |
| | 2 | No dosing | + |
| **2** | 9 | No dosing | + |
| | 16 | No dosing | + |
| **3** | 22 | Test articles, vehicle | |
| | 23 | No dosing | + |
| **4** | 30 | No dosing | + |
| **5** | 37 | No dosing | + |
| **6** | 43 | Test articles, vehicle | |
| | 44 | No dosing | + |
| **7** | 51 | No dosing | + |
| **8** | 58 | No dosing | + |
| **9** | 65 | No dosing | + |
| **10** | 72 | No dosing | + |
| **11** | 79 | No dosing | + |
| **12** | 86 | No dosing | + |

Each serum sample was assayed for the generation by the treatment of an antibody response by titration of anti-angiotensin peptide-antibodies present in the sera by Enzyme Linked ImmunoSorbant Assay (ELISA).

This assay was performed as follows:

Coat the 96 well uniwell microtitre plates with 50 µl detection substrate e.g. Angiotensin II-Gly-Cys-BSA (10 µg peptide equivalent/well) for 1 hour at room temperature. At the same time place 50 µl PBS into separate wells to act as a substrate blank.

Wash the plates 3 times with 200 µl Phosphate Buffered Saline (PBS)/0.1% Tween 20.
Add 200 µl/well of 3% (w/v) milk powder (Marvel) in PBS and leave for 1 hour at room temperature to block nonspecific antibody binding.
Wash the plates 3 times with 200 µl PBS/ 0.1% Tween 20.
Dilute the serum samples to a suitable dilution with PBS. Typical dilutions would be as follows:

i) 1/100 - 5 µl rats sera + 495 µl PBS
ii) 1/1000 - 20 µl (i) + 180 µl PBS
iii) 1/2000 - 10 µl (i) + 190 µl PBS
iv) 1/5000 - 4 µl (i) + 196 µl PBS

Load the appropriate diluted sera (50 µl) to appropriate wells and incubate at 20°C for 1 hour to permit substrate:antibody binding.
Wash the plates 3 times with 200 µl PBS/0.1% Tween 20.
Dilute rabbit anti-rat IgG peroxidase conjugate 1:5000 in PBS i.e. 1 µl IgG peroxidase + 5 mls PBS. This binds to the rat serum antibody and allows antibody detection.
Add 50 µl of the diluted IgG peroxidase to the appropriate wells and leave for 45 minutes at room temperature.
Wash the plates 3 times with 200 µl PBS.
250- µl aliquot of the perodidase substrate $3,3^1$ $,5,5^1$,-tetra methyl benzidine (TMB) to 25 mls 0.1M sodium acetate buffer pH5.5 with 4 µl 30% hydrogen peroxide.
Add 100 µl of the prepared TMB substrate to the appropriate wells, including the blank wells. A colour producing reaction occurs where antibody/ substrate binding has occurred. Leave for 15 minutes at room temperature, then terminate the reaction with 50 µl 10% sulphuric acid added to each well.
The plate was read for absorbance of light at 405 nm generated by the reaction of the peroxidase enzyme on the TMB substrate and is proportional to the amount of primary (anti-angiotensin) antibody bond. Results for the 4 sample conjugate formulations of derivatives (1) to (4) of Example 1 are shown in Figure 1.
Figure 1 shows a time course of mean antibody titre (+/-Sem, n=6) on the y axis at different sample times, measured in days on the x axis. The titre is the SAS estimated dilution of serum required for a 0.1-OD change from baseline levels in the ELISA assay.
The changes in antibody levels against angiotensin peptides can be seen over time, and are summarised below:

| Immunogen | Peak titre | Day | Terminal titre (Day 86) |
|---|---|---|---|
| (B) N-acetyl-cys-gly-Angiotensin I | 12,218 ± 3576 | 86 | As peak |
| (C) Angiotensin I-gly-cys | 9,535 ± 4423 | 30 | 5068 ± 2038 |
| (D) N-acetyl-cys-gly-Angiotensin II | 15,726 ± 8271 | 30 | 10,239 ± 6544 |
| (E) Angiotensin II-gly-cys | 5090 ± 2965 | 37 | 2011 ± 1250 |
| (A) is the control | | | |

In parallel with the antibody titre data, all animals were examined for gross physiological changes in body temperature, weight and general appearance, as an overall assessment of toxic or harmful effects.
No adverse effects were recorded on any of the 4 angiotensin immunoconjugate treatment groups, showing that the treatments are effective in generating anti-angiotensin antibodies, without harmful physiological effects in the animals.

**Example 4:** Effects of active immunisation against angiotensin peptides on the pressor effects of exogenous angiotensin I (AI) in conscious rats

In this experiment to demonstrate the potential of active immunisation with angiotensin analogues, certain analogues of angiotensin I (AI) and angiotensin II (AII) were conjugated to carrier proteins which are good immunogens. These immunoconjugates were adjuvanted and shown in immunised rats to generate a strong anti-angiotensin immune

response.

The immunised rats were examined with regard to inhibition of the pressor response to exogenous AI.

**Materials and Methods**

**Angiotensin immunotherapeutic vaccine preparation**

The angiotensin analogues used in this study were:

AI analogue is: N-acetyl-cysteine-glycine-angiotensin I
AII analogue is: N-acetyl-cysteine-glycine-angiotensin II

The analogues of AI and AII were prepared using a Symphony peptide synthesiser (Anachem).

The conjugation carrier proteins, tetanus toxoid (TT) (Chiron Behring, GmbH), keyhole limpet haemocyanin (KLH) (Biosyn, GmbH) and non toxic recombinant diphtheria toxin (DT) (Chiron Behring, GmbH), were activated using a suitable bivalent linker. The 'activated' carrier protein was separated from the excess cross-linker reagent by size exclusion chromatography.

The following conjugates were made

| Sample Group | Conjugate |
|---|---|
| A | Saline control |
| B | AII analogue, TT carrier protein |
| C | AI analogue, TT carrier protein |
| D | AII analogue, DT carrier protein |
| E | AII analogue, KLH carrier protein |
| F | AII analogue, TT carrier protein |
| G | equal mix of AI and AII analogues TT carrier protein |
| H | AII analogue, TT carrier protein |
| J | AI analogue, TT carrier protein |
| K | AII analogue, TT carrier protein |
| L | AI analogue, TT carrier protein |

Key:

| | |
|---|---|
| AI/AII | Peptide analogues of angiotensin hormones |
| TT | Tetanus toxoid |
| DT | non-toxic recombinant Diphtheria toxin |
| KLH | Keyhole Limpet Haemocyanin |

An excess of the AI and/or AII analogues was mixed with the activated carrier proteins and allowed to react, after which AI/AII-carrier protein conjugates were separated from the remaining free analogue by size exclusion chromatography.

The conjugates were sterilised by filtration through a 0.2 µm filter (Millipore) and formulated with adjuvant and saline vehicle to yield the appropriate vaccine for administration.

Alhydrogel® (Superfos S.A.) was the chosen aluminium hydroxide gel adjuvant for this study and 0.9% saline (Flowfusor®, Fresenius) the vaccine vehicle.

Table 2 shows the conjugate formulations administered to each of the treatment groups. The conjugates were formulated with aluminium hydroxide adjuvant, other than the conjugate of Group F which was formulated with DEAE (diethylaminoethyl)-dextran adjuvant.

**Immunisation and AI Challenge**

Male, Sprague Dawley rats (initially 200-250 g: Harlan Olac: n=6 for all groups) were injected (0.5 ml, sc.)

with saline or immunotherapeutic vaccines on the days specified in Table 2.

On day 61, under sodium methohexitone anaesthesia (40-60 mg $kg^{-1}$ i.p., supplemented as required), catheters were implanted in the distal abdominal aorta (via the ventral caudal artery) and right jugular vein. The following day, conscious rats were given increasing i.v. bolus (0.1 ml) doses of AI (3-60 pmol $rat^{-1}$), while mean systemic arterial blood pressure and heart rate were recorded. At the end of the experiment animals were given i.v. sodium pentobarbitone (100 mg) and a blood sample was taken by cardiac puncture for the measurement of anti-angiotensin antibodies by ELISA.

Table 2
**Treatment regime, formulations, doses, injection frequency and experimental regimes for study**

| Group | Formulation | Vol/Dose | Injections, Days 0 | 14 | 21 | 28 | 42 | Catheters, Days 61 | Challenge (AI), Days 62 |
|---|---|---|---|---|---|---|---|---|---|
| A | Saline Control | 0.2 ml | X | | X | | X | X | X |
| B | AII analogue, TT carrier protein, AlOH adjuvant | 5 μg | X | | X | | X | X | X |
| C | AI analogue, TT carrier protein, AlOH adjuvant | 5 μg | X | | X | | X | X | X |
| D | AII analogue, DT carrier protein, AlOH adjuvant | 5 μg | X | | X | | X | X | X |
| E | AII analogue, KLH carrier protein, AlOH adjuvant | 5 μg | X | | X | | X | X | X |
| F | AII analogue, TT carrier protein, DEAE adjuvant | 5 μg | X | | X | | X | X | X |
| G | equal mix of AI and AII analogues TT carrier protein, AlOH adjuvant | 2x2.5 μg | X | | X | | X | X | X |
| H | AII analogue, TT carrier protein, AlOH adjuvant | 25 μg | X | | X | | X | X | X |
| J | AI analogue, TT carrier protein, AlOH adjuvant | 25 μg | X | | X | | X | X | X |
| K | AII analogue, TT carrier protein, AlOH adjuvant | 5 μg | X | X | | X | | X | X |
| L | AI analogue, TT carrier protein, AlOH adjuvant | 5 μg | X | X | | X | | X | X |

Key:

| | |
|---|---|
| AI/AII | Peptide analogues of angiotensin hormones |
| TT | Tetanus toxoid |
| DT | non-toxic recombinant Diphtheria toxin |
| KLH | Keyhole Limpet Haemocyanin |
| DEAE | Diethylaminoethyl cellulose |
| AlOH | Aluminium hydroxide gel |

**Angiotensin analogue antibody ELISA**

ELISA plate wells (Anachem) were coated with 10μg peptide equivalents of either AI or AII conjugated to bovine serum albumin (BSA) as a carrier.

The coated wells were washed with PBS (0.2% w/v)/Tween (Sigma) and blocked with 3% Marvel before diluted sera from the vaccinated rats were incubated in their respective wells. The sera had been diluted in PBS (Sigma) over a range from 2,500-20,000 fold.

Immobilised antibodies were detected in the wells using a rabbit anti-rat IgG/horseradish peroxidase conjugate and revealed using 3,3'-5,5'-tetra-methyl benzidine with $H_2O_2$ (Sigma). The reaction was terminated after 15 min at 22°C by the addition of 10% (v/v) $H_2SO_4$ (Sigma).

Colour generated was determined by absorbance at 450nm using a Packard plate reader. The resultant absorbance readings were analysed by a statistical package (SAS Institute 1997) to determine titre.

**Statistical analysis of blood pressure changes on AI Challenge**

The maximum change in mean blood pressure and heart rate over their immediate pre-challenge values were calculated for each animal and each challenge dose. Differences between treated groups and unimmunised controls were assessed by ANOVA using Dunnett's test.

**Dose response analysis**

The main effect of immunisation was to cause a parallel shift in the blood pressure dose response of animals to AI challenge. To estimate the size of this shift, a logistic model was derived and fitted to the dose response:

$$\Delta BP = \frac{\Delta BP_{\max}}{1 + (d\ /\mathrm{ED50})^{-\alpha}} + \varepsilon \qquad \varepsilon \sim N(0,\sigma^2)$$

where $d$ is the dose of AI, $BP_{max}$ is the maximal change in blood pressure, $\alpha$ a shape parameter and $ED_{50}$ is the dose of AI giving a half maximal response. Separate $ED_{50}$ estimates were obtained for each animal. Significant differences between treatment groups and unimmunised controls were assessed by ANOVA of log-transformed $ED_{50}$ values using Dunnett's multiple comparison test.

**Results**

Table 3 summarises some of the results, showing that active immunisation caused significant shifts in the pressor dose-response to AI and marked increases in antibody titres.

Clear effects on blood pressure are demonstrated with these treatments and the maximum dose shift (8.9x the control) are seen with a conjugate containing the AI analogue and tetanus toxoid on an aluminium hydroxide adjuvant.

Table 3 also demonstrates the relationship between anti-angiotensin antibody titre and response. In general, it can be seen that there is broad agreement between treatment induced titre and mean treatment induced dose shift, but no obvious dose response between groups C and J is apparent.

Figures 2 and 3 illustrate the results for control rats (Group A) and rats immunised with a conjugate of the AI analogue and tetanus toxoid, presented on an AlOH gel adjuvant at a peptide equivalent dose of 5μg (low; Group C) and 25μg (high; Group J).

Figure 2 is a graph showing pressor effects of AI in control rats (Group A) and rats immunised with AI analogue at a dose of 5 μg (low, Group C) or 25 μg (High, Group J). The y axis shows peak change in BP (mm Hg) and the x axis shows the angiotensin I dose (pmol/rat) in control, high dose group J (25 μg) and low dose group C (5 μg) animals. Errors bars shows 95% confidence interval on mean, based on pooled within group standard deviation (n=6), shown for control group only.

Figure 3 shows recordings of mean blood pressure changes in response to AI (3, 18 and 60 pmol bolus dose) in representative animals from group A(control) and Group C (5 μg dose).

**Conclusion**

Treatment with a conjugate containing an AI analogue and tetanus toxoid on an aluminium hydroxide adjuvant gives a highly significant reduction in the pressor response to exogenous AI.

Table 3

| Treatment | Median $ED_{50}$ | Mean treatment-induced dose shift | Anti-angiotensin antibody titre, ± s.e. mean (n=6) |
|---|---|---|---|
| A | 8.9 | - | 0 0 |
| B | 39.6 | 4.5* | 15300 ± 2100 |
| C | 79.1 | 8.9*** | 32100 ± 7800 |
| D | 19.6 | 2.2 | 9200 ± 2200 |
| E | 17.6 | 2.0 | 4700 ± 600 |
| F | 15.2 | 1.7 | 5500 ± 700 |
| G | 24.5 | 2.8 | 8300 ± 2000 |
| H | 38.2 | 4.3* | 12100 ± 2500 |
| J | 74.7 | 8.4*** | 20100 ± 2300 |
| K | 13.9 | 1.6 | 5000 ± 900 |
| L | 43.0 | 4.8* | 26100 ± 9400 |

Median AI bolus (pmol.$rat^{-1}$) to achieve half-maximal increase in mean blood pressure ($ED_{50}$) and corresponding anti-angiotensin antibody titres in control (group A) and immunised (groups B-L) rats. Significance probabilities adjusted for multiple comparisons by Dunnett's method (*=P<0.05, **=P<0.01, ***=P<0.001).

**Example 5:** Characterisation of antibodies produced in Example 3

Antibodies produced in Example 3 were enriched by affinity chromatography as follows:

**Materials**

1 mL HiTrap protein G affinity column (Pharmacia Biotech: 17-0404-03)

Wash buffer (WB) = PBS pH 7.2
Elution buffer (EB) = 0.1M glycine (HCL) pH 2.7
Neutralizing buffer (NB) = 1M Tris (HCL) pH 9
Storage buffer (SB) = 20% ethanol (v/v)

1. Rat sera from terminal bleeds following inoculation with each of TT-NAc-CG-angiotensin I (5 mL), angiotensin I-GC-TT (7 mL), TT-NAc-CG-angiotensin II (6 mL) or angiotensin II-GC-TT (5 mL), were clarified by centrifugation, filtered through a 1 μm PTFE disc filter then dialyzed against PBS pH 7.2. Each was then separately enriched as follows.

2. The HiTrap column was washed and equilibrated with 5 mL of WB.

3. Prepared sera (Point 1) was passed once through the HiTrap column, the waste was collected and stored at -20°C.

4. The HiTrap column was washed with 5 mL of WB to remove any remaining waste sera.

5. Immobilized antibodies were eluted using 10 mL of EB. The eluent was collected in 1 ml fractions each being immediately neutralized with 0.1 mL of NB.

6. At the end of the run the HiTrap column was washed with SB and stored at 4°C.

ELISA assays were carried out as described in Example 3 using plates coated as follows:

1) for angiotensin I and II

**Materials:**

Nunc Maxisorp ELISA plates (Life Technologies: 430341A).
Human Angiotensin I (Bachem: H-1680)
Human Angiotensin II (Bachem: H-1705)
0.1M carbonate buffer, pH 9.8 (0.316g $Na_2CO_3$ & 0.584g $NaHCO_3$ per 0.1L)

Other materials used were as in the ELISA method detailed in Example 3 above.

1. 100 µL of either angiotensin I or angiotensin II, depending on the specific binding event to be measured @ 0.2 mg/ml of 0.1M carbonate buffer, pH 9.8) was added to a suitable number of ELISA plate wells, and incubated for 1 hour at 22°C.

2. The ELISA plate was washed with PBS/Tween, blocked with Marvel then washed with PBS/Tween again as by the ELISA method described in Example 3 above.

3. The enriched antibodies from sera raised to Tetanus toxoid (TT) conjugates TT-NAc-CG-Ang I, Ang I-GC-TT, TT-NAc-CG-Ang II and Ang II-GC-TT, were incubated (1 hour, 22°C) in the coated wells at 2.5 µg/ml of PBS pH 7.2.

The ELISA was then completed as by the method described in Example 3 but absorbance was read at 450 nm.

2) For angiotensinogen:

The ELISA for detection of native angiotensinogen (Sigma: A-2562) was performed by the method of Example 3.

The enriched antibodies from sera raised to Tetanus Toxoid (TT) conjugates TT-NAc-CG-Ang I, Ang I-GC-TT, TT-NAc-CG-Ang II and Ang II-GC-TT were incubated (1 hour 22°C) at 0.5 µg/ml of PBS pH 7.2.

Results are shown in Figures 4, 5 and 6 which show absorbance read with ELISA plates coated with angiotensin I (Fig. 4), angiotensin II (Fig. 5) and angiotensinogen (Fig. 6) and shows that antibodies raised to each of the angiotensin derivatives conjugated to TT ie TT-NAc-CG-Ang I, Ang I-GC-TT, TT-NAc-CG-Ang II and Ang II-GC-TT recognised angiotensin I, angiotensin II and angiotensinogen.

**Example 6:** Generation of further angiotensin derivatives and immunisation studies

The following angiotensin derivative peptides 1 - 6 were synthesised according to the method of Example 1

N-acetyl-Cys-Ala-Angiotensin I (1)

N-acetyl-Cys-$(Ala)_4$-Angiotensin I (2)

N-acetyl-Cys$(Gly)_6$-Angiotensin I (3)

N-acetyl-Cys-Gly-Ala-Gly-Ala-Angiotensin I (4)

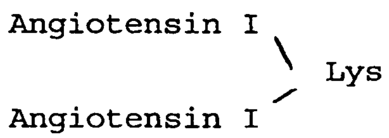

(5)

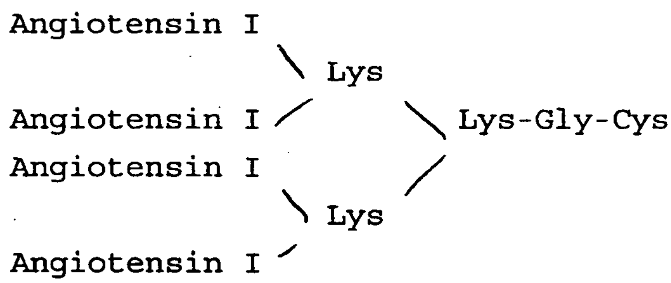

(6)

These peptides were conjugated to tetanus toxoid as described in Example 2, and formulated for immunisation studies using the protocol described in Example 3.

Antibody titres were measured using the ELISA technique described in Example 4 against the peptides used in the immunogen.

Results are shown in Figures 7 and 8 which are graphs showing, on the y axis the antibody titre, as the dilution factor to produce a SAS™ (Statistics Analysis System) designated 0.1 OD unit change, at various sample days. Each data point shown represents the mean of 5 serum samples from 5 different animals, each assayed in duplicate.

All peptides were shown to be effective in generating an anti-angiotensin I response. The responses varied in extent and duration.

**Claims**

1. An immunogenic angiotensin derivative comprising at least one angiotensin peptide moiety of sequence Asp-Arg-Val-Tyr-Ile-His-Pro-Phe-His-Leu coupled via a peptide carrier-binding moiety to a carrier.

2. An angiotensin derivative as claimed in claim 1 wherein the carrier binding moiety contains the residue of an amino acid having a reactive side chain.

3. An angiotensin derivative as claimed in claim 1 or claim 2 wherein the carrier binding moiety is a peptide extension at the N- or the C-terminus of an angiotensin peptide moiety.

4. An angiotensin derivative as claimed in any one of claims 1 to 3 wherein said carrier is a polypeptide.

5. An angiotensin derivative as claimed in any one of claims 1 to 4 wherein the carrier is selected from the purified protein derivative of tuberculin, tetanus toxoid, diphtheria toxoid, keyhole limpet haemocyanin or derivatives thereof.

6. An angiotensin derivative as claimed in any one of claims 1 to 5 wherein said peptide carrier-binding moiety has the sequence Gly-Cys whereby said derivative comprises a peptide moiety of sequence Asp-Arg-Val-Tyr-Ile-His-Pro-Phe-His-Leu-Gly-Cys, coupled to a carrier.

7. An angiotensin derivative as claimed in any one of claims 1 to 6 which elicits a cross-reactive immune response with angiotensin I, angiotensin II, and/or angiotensinogen molecules.

8. The use of an immunogenic angiotensin derivative comprising at least one angiotensin moiety coupled via a peptide carrier-binding moiety to a carrier in the manufacture of a medicament for use in combatting diseases associated

with the renin-angiotensin system.

**9.** The use as claimed in claim 8 wherein the angiotensin moiety comprises angiotensin I or angiotensin II or a functional equivalent of angiotensin I or angiotensin II.

**10.** The use as claimed in claim 8 or claim 9 wherein the carrier binding moiety contains an amino acid residue having a reactive side chain.

**11.** The use as claimed in any one of claims 8 to 10 wherein the carrier binding moiety is a peptide extension at the N- or the C-terminus of an angiotensin peptide moiety.

**12.** The use as claimed in any one of claims 8 to 11 wherein the angiotensin derivative is selected from

(A)-Gly Cys

(A)-Cys

(A)-Tyr

N-acetyl-Cys-(A)

Tyr-(A)

N-acetyl-Cys-Gly-(A)

Cys - (A)

(A) - N-acetyl-Cys

where A is angiotensin I or II.

**13.** The use as claimed in any one of claims 8 to 12 wherein the angiotensin derivative elicits a cross-reactive immune response with angiotensin I, angiotensin II, and/or angiotensinogen molecules.

**14.** The use as claimed in any one of claims 8 to 13 wherein said carrier is a polypeptide.

**15.** The use as claimed in any one of claims 8 to 14 wherein the carrier is selected from the purified protein derivative of tuberculin, tetanus toxoid, diphtheria toxoid, keyhole limpet haemocyanin or derivatives thereof.

**16.** The use as claimed in any one of claims 8 to 15 wherein said disease is congestive heart failure or hypertension.

**17.** The use as claimed in any one of claims 8 to 15 for the modulation of blood pressure.

**18.** A pharmaceutical composition comprising an angiotensin derivative as defined in any one of claims 1 to 17, together with one or more pharmaceutically acceptable carriers or excipients.

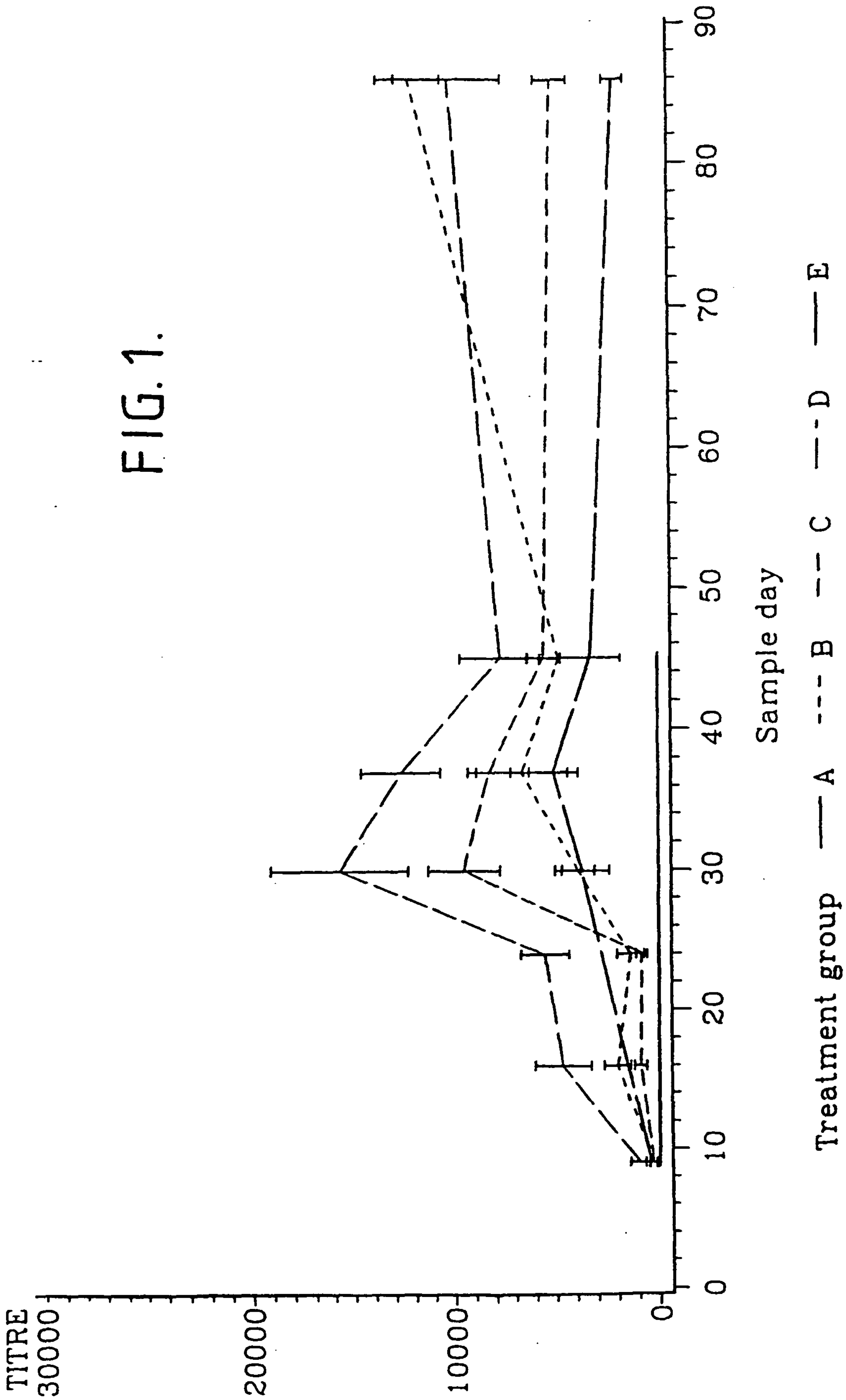
TITRE
30000
20000
10000
0
0
10
20
30
40
50
60
70
80
90
Sample day
Treatment group — A --- B — — C — -D — E

FIG. 1.

FIG. 2.

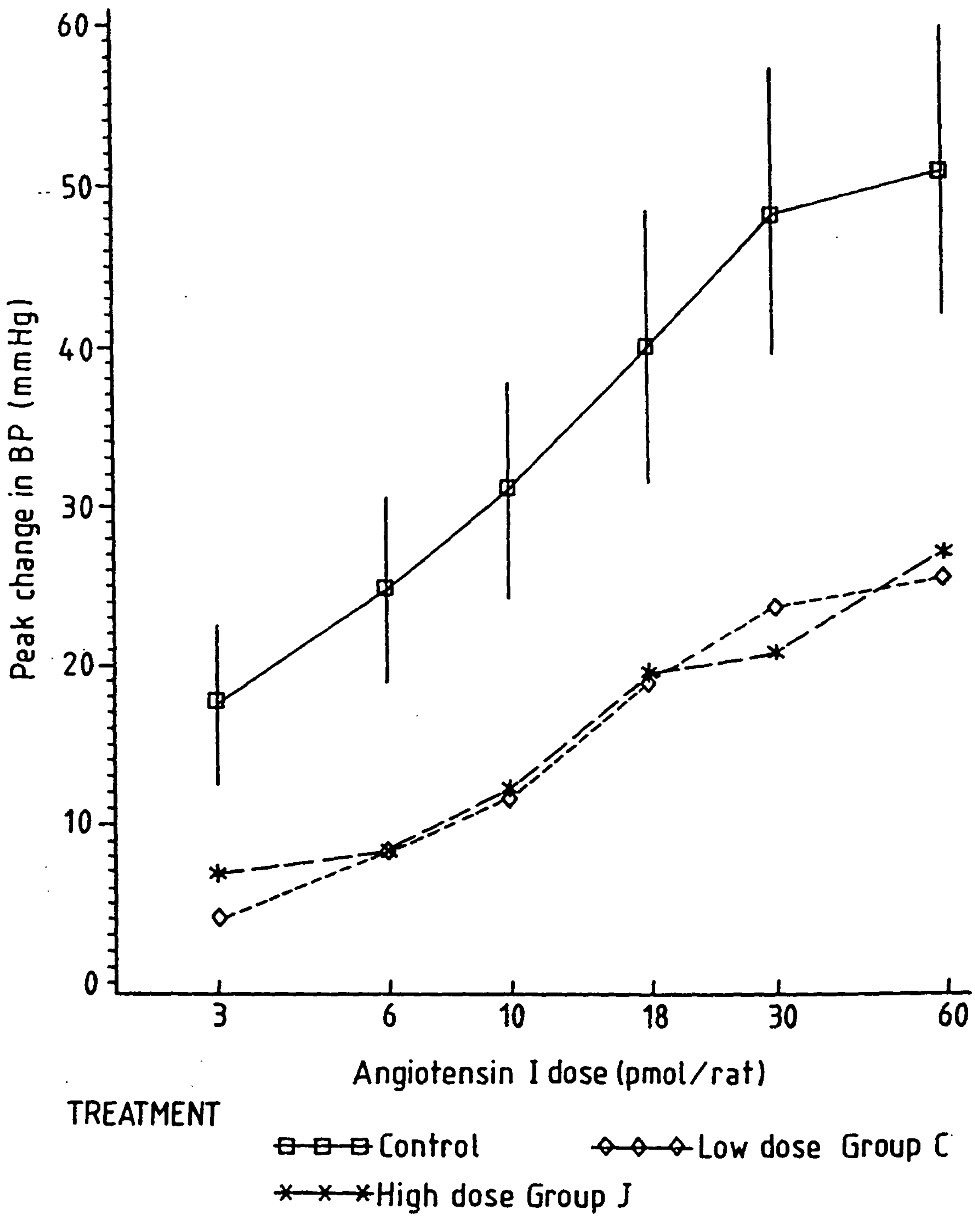
60
50
40
30
20
10
0
Peak change in BP (mmHg)
3
6
10
18
30
60
Angiotensin I dose (pmol/rat)
TREATMENT
Control
Low dose Group C
High dose Group J

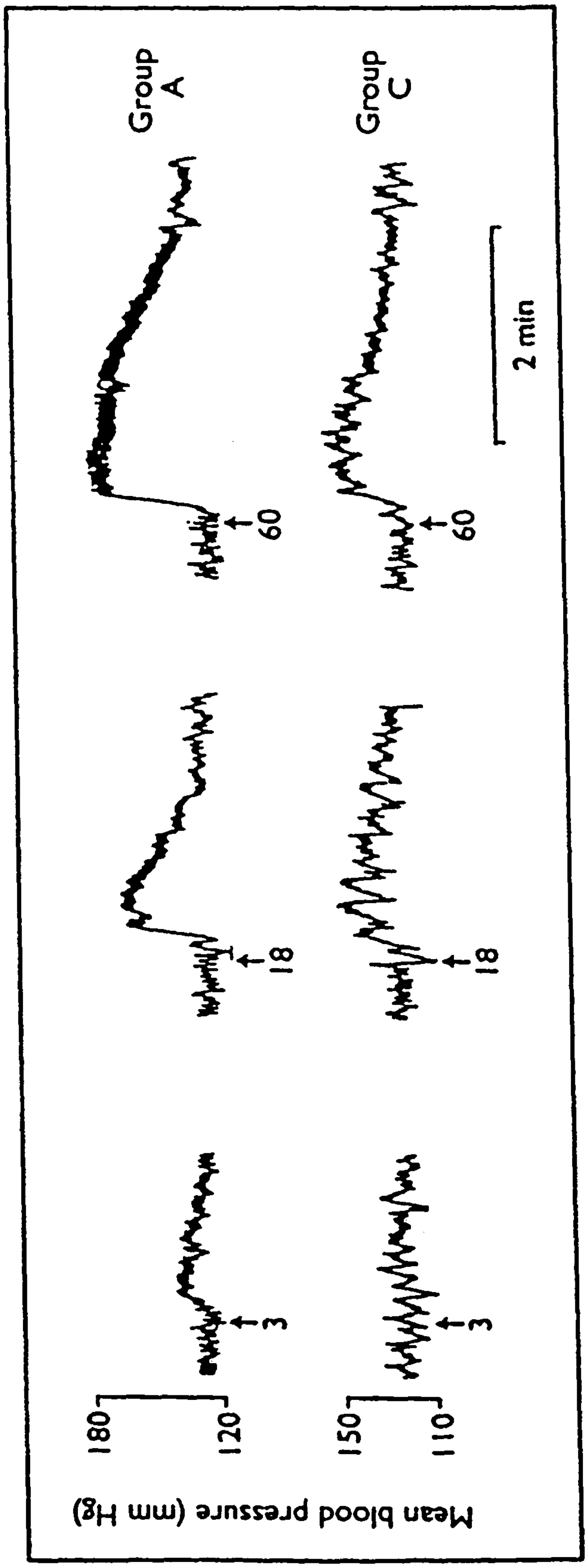
Mean blood pressure (mm Hg)
180
120
150
110
3
18
60
Group A
Group C
2 min

FIG. 3.

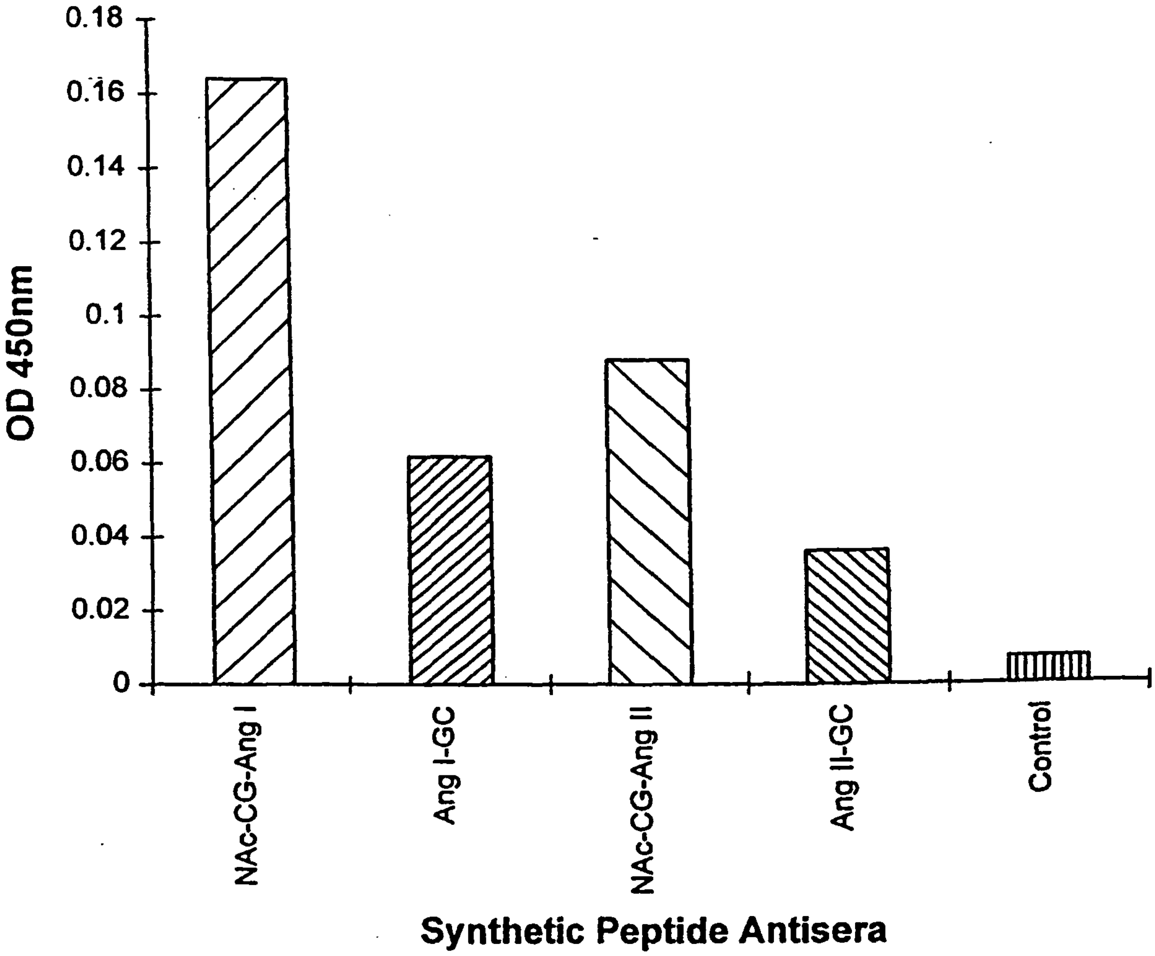
0.18
0.16
0.14
0.12
0.1
0.08
0.06
0.04
0.02
0
OD 450nm
NAc-CG-Ang I
Ang I-GC
NAc-CG-Ang II
Ang II-GC
Control
Synthetic Peptide Antisera

FIG. 4.

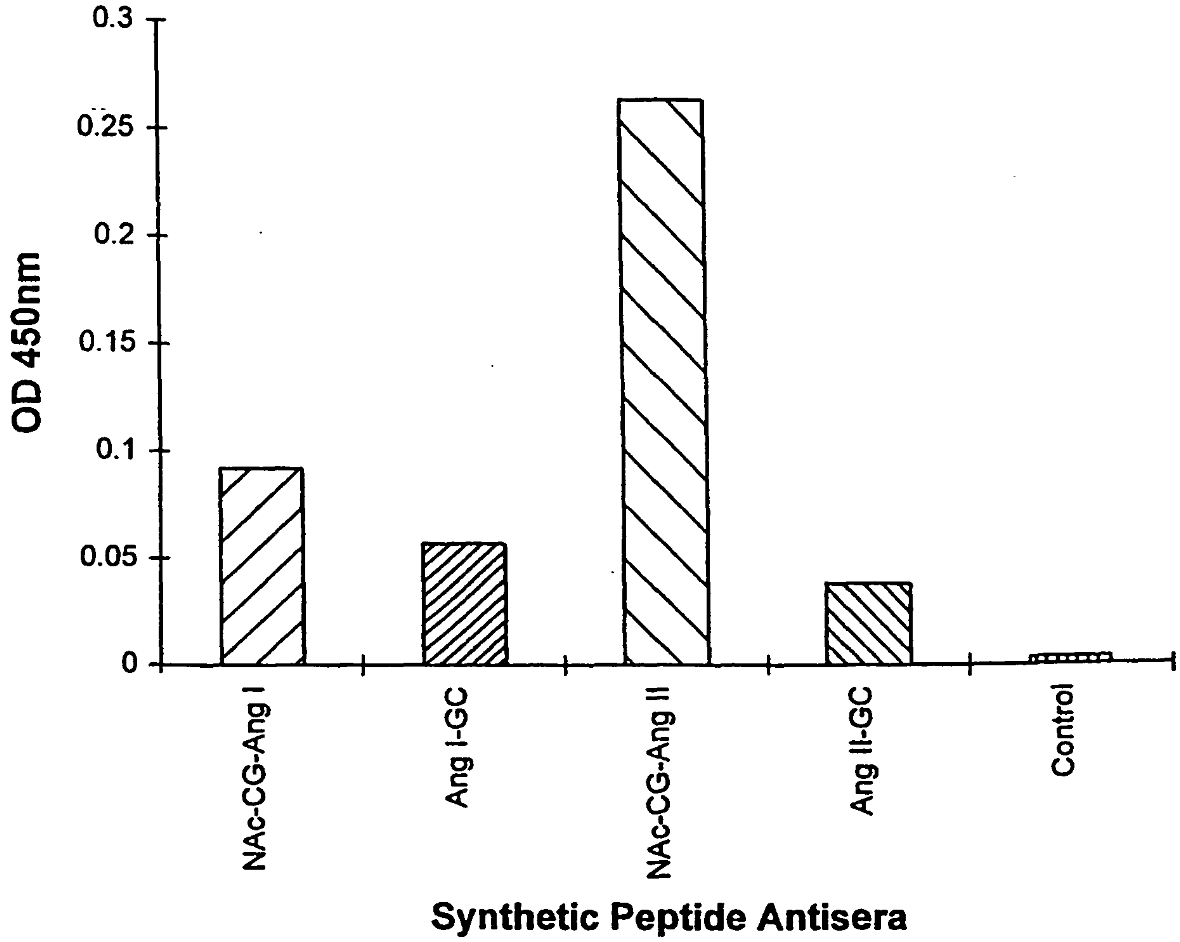
0.3
0.25
0.2
0.15
0.1
0.05
0
OD 450nm
NAc-CG-Ang I
Ang I-GC
NAc-CG-Ang II
Ang II-GC
Control
Synthetic Peptide Antisera

FIG. 5.

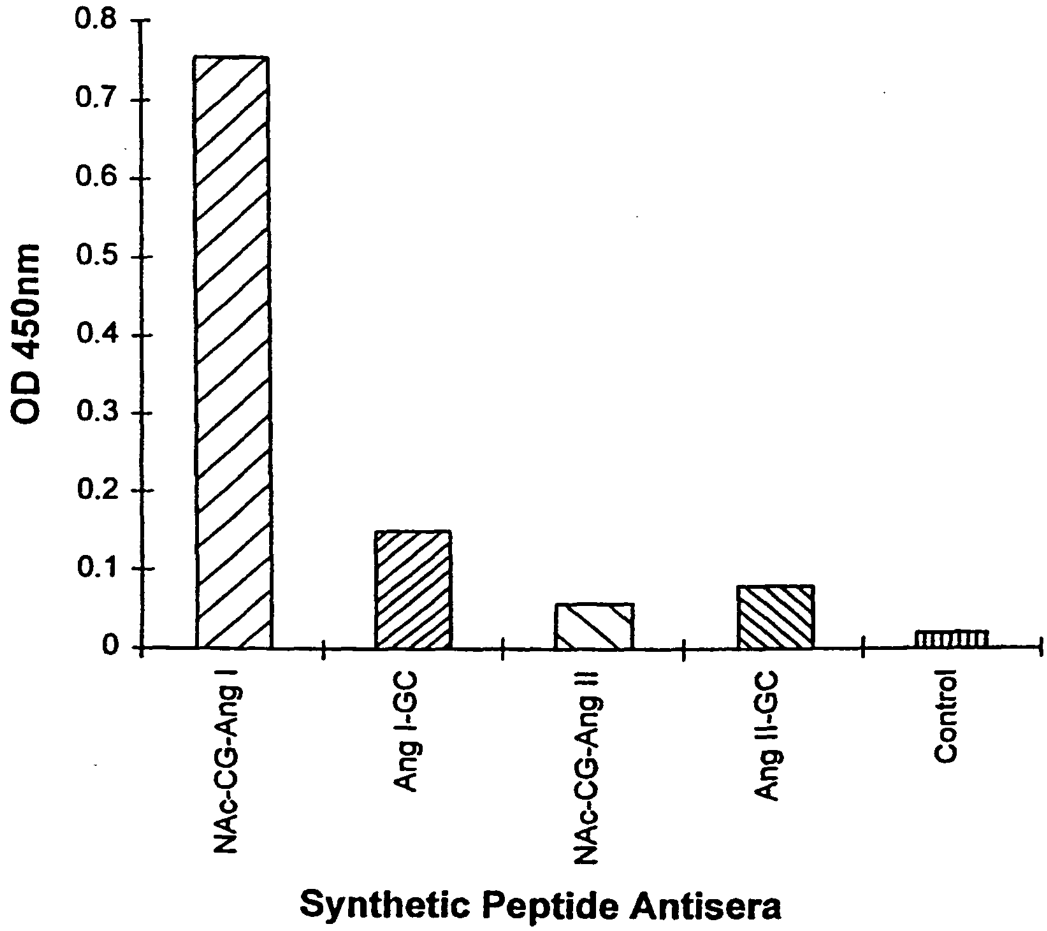
0.8
0.7
0.6
0.5
0.4
0.3
0.2
0.1
0
OD 450nm
NAc-CG-Ang I
Ang I-GC
NAc-CG-Ang II
Ang II-GC
Control
Synthetic Peptide Antisera

FIG. 6.

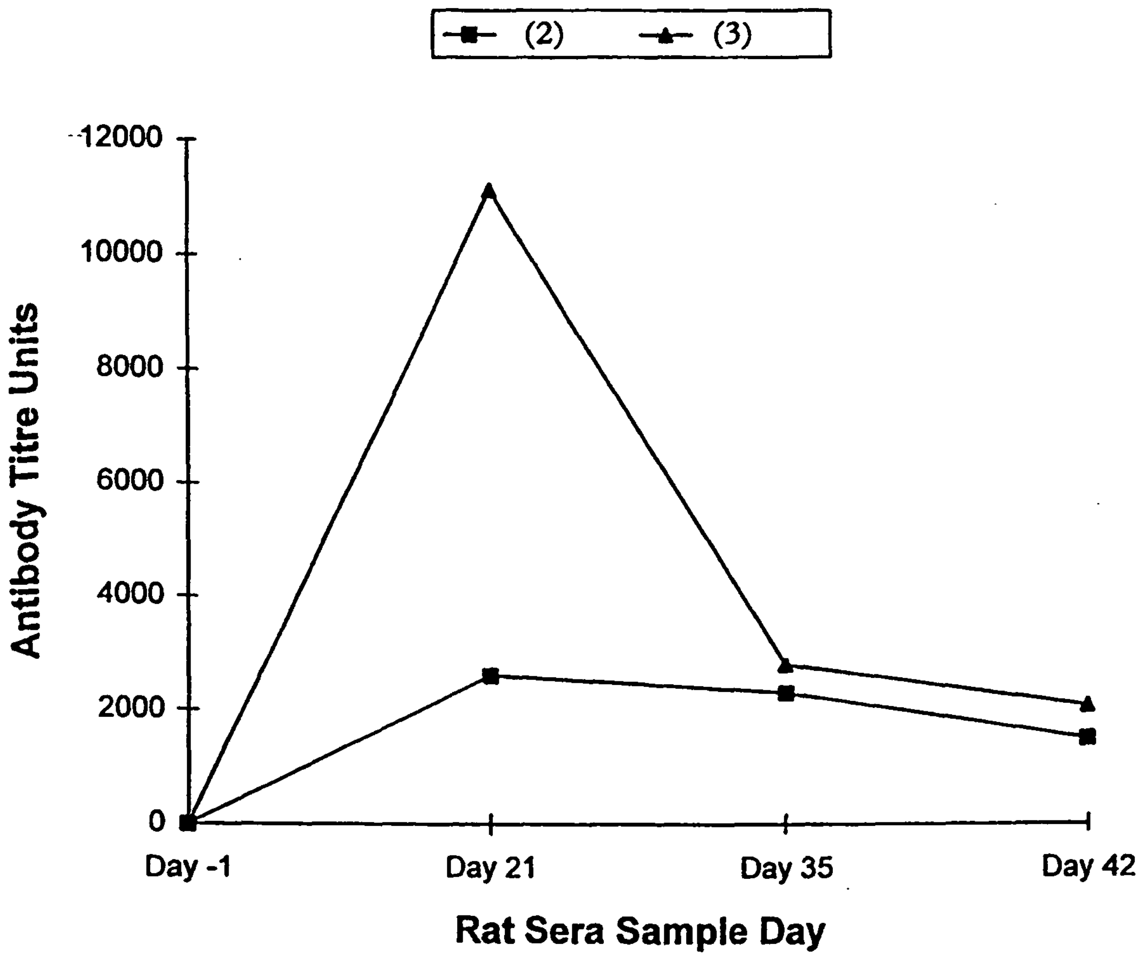
(2)
(3)
Antibody Titre Units
12000
10000
8000
6000
4000
2000
0
Day -1
Day 21
Day 35
Day 42
Rat Sera Sample Day

FIG. 7.

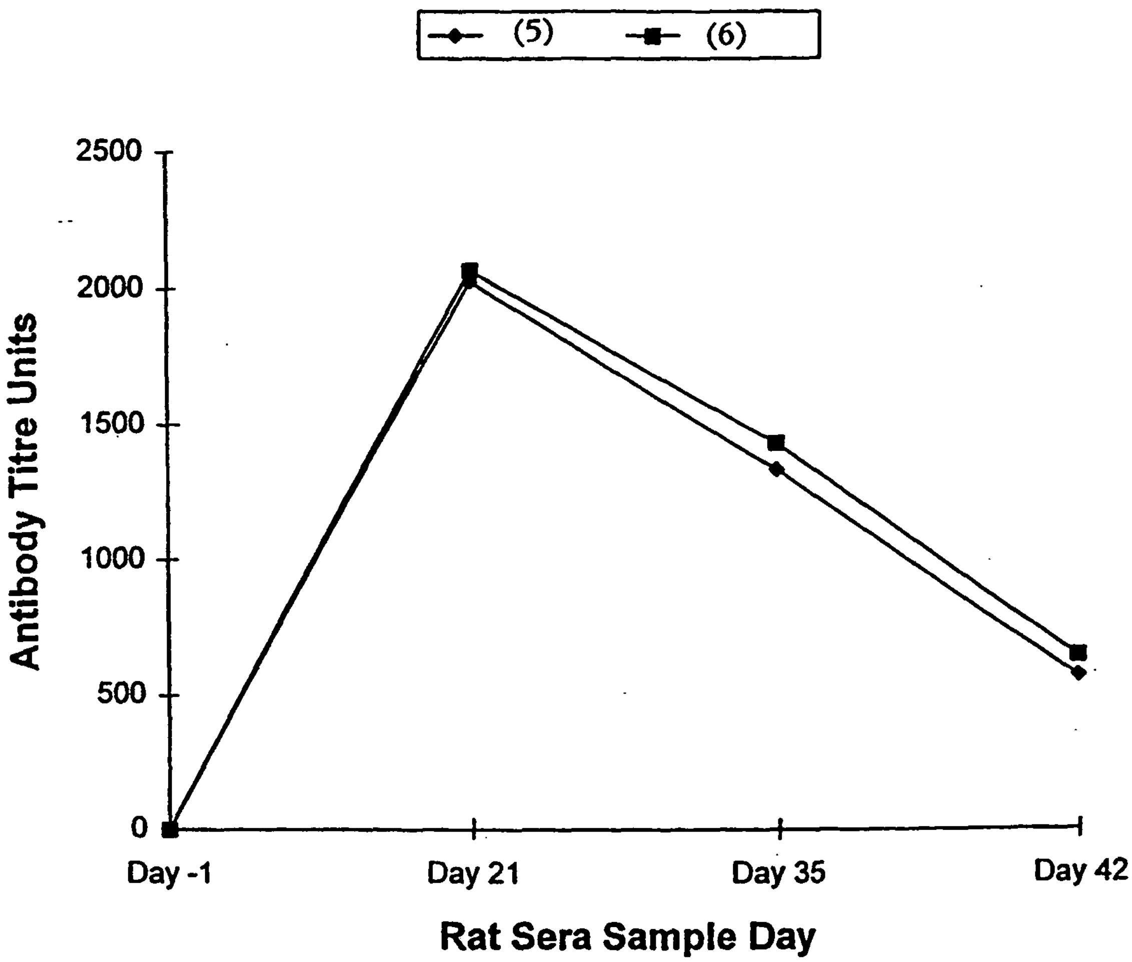
(5)
(6)
2500
2000
1500
1000
500
0
Antibody Titre Units
Day -1
Day 21
Day 35
Day 42
Rat Sera Sample Day

FIG. 8.

European Patent Office

# EUROPEAN SEARCH REPORT

Application Number
EP 04 01 6314

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 384 995 A (STEVENS VERNON C) 24 May 1983 (1983-05-24)<br>* column 6, line 60 *<br>* column 48, last paragraph *<br>----- | 1-18 | C07K7/14<br>C07K14/00<br>A61K47/48<br>C12N15/16 |
| X | J.M PEETERS ET AL.: "Comparison of four bifunctional reagents for coupling peptides to proteins..." J. IMMUNOLOGICAL METHODS, vol. 120, 1989, pages 133-143, XP002080173<br>* abstract *<br>* page 135, column 1, last paragraph; figure 1 *<br>----- | 1-18 | |
| X | WO 93/08842 A (SOMATOGEN INC) 13 May 1993 (1993-05-13)<br>* example 1 *<br>----- | 1-18 | |
| X | K.Y. KUMAGAYE ET AL.: "Suppression of a side reaction associated with Nim-benzyloxymethyl group during synthesis of peptides containing cysteinyl residue at the N-terminus" PEPTIDE RESEARCH, vol. 4, no. 2, 1991, pages 84-87, XP002080174<br>* page 84, column 1, paragraph 1 *<br>----- | 1-18 | TECHNICAL FIELDS SEARCHED (Int.Cl.7)<br>C07K<br>A61K<br>C12N |
| X | KAWABE H ET AL: "CHARACTERIZATION OF RECEPTORS FOR ANGIOTENSIN-INDUCED DRINKING AND BLOOD PRESSURE RESPONSES IN CONSCIUS RATS USING ANGIOTENSIN ANALOGSEXTENDED AT THE N-TERMINAL" NEUROENDOCRINOLOGY, vol. 42, 1986, pages 289-295, XP002058941<br>* abstract; table 1 *<br>-----<br>-/-- | 1-18 | |

The present search report has been drawn up for all claims

| Place of search | Date of completion of the search | Examiner |
|---|---|---|
| Munich | 19 October 2004 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 04 01 6314

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | F.S. TJOENG ET AL.: "Multiple peptide synthesis using a single support (MPS3)" INTERNATIONAL JOURNAL OF PEPTIDE AND PROTEIN RESEARCH, vol. 35, 1990, pages 141-146, XP002080175 COPENHAGEN DK * the whole document * ----- | | |
| A | US 5 229 490 A (TAM JAMES P) 20 July 1993 (1993-07-20) * the whole document * ----- | | |

TECHNICAL FIELDS SEARCHED (Int.Cl.7)

The present search report has been drawn up for all claims

| Place of search | Date of completion of the search | Examiner |
|---|---|---|
| Munich | 19 October 2004 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 01 6314

*This annex lists the patent family members relating to the patent documents cited in the above-mentioned European search report.*
The members are as contained in the European Patent Office EDP file on
The European Patent Office is in no way liable for these particulars which are merely given for the purpose of information.

19-10-2004

| Patent document cited in search report | Publication date | Patent family member(s) | Publication date |
|---|---|---|---|
| US 4384995 A | 24-05-1983 | US 4302386 A | 24-11-1981 |
| | | CA 1223206 A1 | 23-06-1987 |
| | | US 6096318 A | 01-08-2000 |
| | | US 6146633 A | 14-11-2000 |
| | | US 5698201 A | 16-12-1997 |
| | | US 6039948 A | 21-03-2000 |
| | | US 6143305 A | 07-11-2000 |
| | | US 4526716 A | 02-07-1985 |
| | | US 4762913 A | 09-08-1988 |
| WO 9308842 A | 13-05-1993 | US 5545727 A | 13-08-1996 |
| | | AT 168014 T | 15-07-1998 |
| | | AU 665599 B2 | 11-01-1996 |
| | | AU 3132493 A | 07-06-1993 |
| | | CA 2122717 A1 | 13-05-1993 |
| | | DE 69226197 D1 | 13-08-1998 |
| | | DE 69226197 T2 | 11-02-1999 |
| | | EP 0611306 A1 | 24-08-1994 |
| | | JP 7500840 T | 26-01-1995 |
| | | JP 3426599 B2 | 14-07-2003 |
| | | WO 9308842 A1 | 13-05-1993 |
| | | US 5679777 A | 21-10-1997 |
| | | US 5759517 A | 02-06-1998 |
| | | AT 167485 T | 15-07-1998 |
| | | AU 672960 B2 | 24-10-1996 |
| | | AU 3133793 A | 07-06-1993 |
| | | CA 2121889 A1 | 13-05-1993 |
| | | DE 69225978 D1 | 23-07-1998 |
| | | DE 69225978 T2 | 11-02-1999 |
| | | EP 0611376 A1 | 24-08-1994 |
| | | EP 0857489 A2 | 12-08-1998 |
| | | FI 942138 A | 29-06-1994 |
| | | HU 70309 A2 | 28-09-1995 |
| | | JP 7501059 T | 02-02-1995 |
| | | NO 941703 A | 04-07-1994 |
| | | SG 47882 A1 | 17-04-1998 |
| | | US 6184356 B1 | 06-02-2001 |
| | | WO 9309143 A1 | 13-05-1993 |
| | | US 5599907 A | 04-02-1997 |
| | | US 5739011 A | 14-04-1998 |
| | | US 6274331 B1 | 14-08-2001 |
| | | US 5801019 A | 01-09-1998 |
| | | US 5744329 A | 28-04-1998 |
| | | US 5844088 A | 01-12-1998 |
| | | US 5798227 A | 25-08-1998 |
| | | US 5844089 A | 01-12-1998 |



For more details about this annex : see Official Journal of the European Patent Office, No. 12/82

## ANNEX TO THE EUROPEAN SEARCH REPORT ON EUROPEAN PATENT APPLICATION NO. EP 04 01 6314

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2004

| Patent document cited in search report | Publication date | Patent family member(s) | Publication date |
|---|---|---|---|
| WO 9308842 A | | US 6150506 A | 21-11-2000 |
| US 5229490 A | 20-07-1993 | NONE | |

EPO FORM P0459

For more details about this annex : see Official Journal of the European Patent Office, No. 12/82